# EUROPEAN PATENT SPECIFICATION

(11) **EP 0 837 636 B1**
(45) Date of publication and mention of the grant of the patent: **29.08.2001**
(21) Application number: 96920918.8
(22) Date of filing: 14.06.1996
(51) Int. Cl.: A23K 1/14, A23K 1/16, A23K 1/18, A61K 35/78

(54) **ANIMAL FEED**
TIERFUTTER
ALIMENTS POUR ANIMAUX

(30) Priority: 14.06.1995 GB 9512106
(43) Date of publication of application: 29.04.1998
(73) Proprietor: MATFORSK, Norwegian Food Research Institute, 1430 Äs (NO)
(72) Inventor: SLINDE, Erik, N-5030 Land s (NO); SAHLSTROM, Stefan, N-1430 s (NO); FARDAL, Leif, Arne, N-1440 Dr bak (NO); HOLCK, Askild, N-1430 s (NO); SKREDE, Grete, N-1430 s (NO)
(74) Representative: Cockbain, Julian, Dr.
(86) International application number: GB9601414
(87) International publication number: WO9700018

(56) References cited:
- EP-A- 0 137 869
- EP-A- 0 162 805
- WO-A-91/09132
- WO-A-91/13076
- WO-A-93/18179
- WO-A-94/01122
- FR-A- 2 695 649
- GB-A- 1 366 095
- DATABASE WPI Week 8223 Derwent Publications Ltd., London, GB; AN 82-47164E XP002012288 & JP,A,57 071 371 (TSUNEKAWA F) , 4 May 1982 & PATENT ABSTRACTS OF JAPAN vol. 6, no. 149 (C-118), 10 August 1982 & JP,A,57 071371 (TSUMEKAWA FUJIO), 4 May 1982,
- PATENT ABSTRACTS OF JAPAN vol. 17, no. 674 (C-1140), 10 December 1993 & JP,A,05 221874 (MITSUBISHI PETROCHEM CO LTD), 31 August 1993,
- APPLIED AND ENVIRONMENTAL MICROBIOLOGY, vol. 55, no. 8, 1989, US, pages 1901-1906, XP002012287 U. SCHILLINGER ET AL.: "Antibacterial activity of Lactobacillus sake isolated from meat"

## Description

The present invention relates to animal feed and to a process for preparing animal feed which yields improved feed quality and digestibility.

Carbohydrates and starch are used as feedstuffs in the farming industry and in this regard cereals, such as for example oats, used as a component of fish feed in the fish farming industry are a well-known carbohydrate source for farmed salmonids. Studies have shown that salmonids are capable of digesting starch from cereals and that cereals can be mixed in fish feed without adverse effects on digestibility or on the performance of the fish. However, in general, it is observed that the higher the level of cereal present in the feed, the lower the level of digestibility. As a typical example, an increase from 10% to 30% by weight of oats in fish feed typically reduces starch digestibility from about 61% to about 33%. Thus there is generally a need to increase starch digestibility in fish feed.

However, to date only small quantities of cereals have been used as a component of fish feed because of difficulty in extruding feed mixtures into pellets. Conventional feed pellets are also prone to contamination by bacterial strains such as Listeria monocytogenes. This poses a significant problem in the fish farming industry.

We have now found that pre-fermenting of cereal facilitates formation of firm feed pellets and produces feed which is more digestible than conventional feed containing non-fermented cereal, and moreover that where pre-fermentation is effected using a bacteriocin producing strain of lactic acid bacteria (lactobacilli), the growth of undesirable bacterial strains such as food-borne pathogens or food spoilage bacteria, (for example species of Listeria, eg. Listeria monocytogenes) during the production and storage of the feed is suppressed. Furthermore, fish feed containing prefermented cereal has also been found to exhibit an immunostimulating effect.

Bacteriocins are peptides or proteins released by bacteria which show bactericidal or bacteriostatic activity towards both the producing strain and/or other bacteria. Generally, only bacteria closely related to the producing strain are inhibited although in some cases other, non related bacteria, including certain pathogens may also be affected.

Due to their potential use as antibacterial agents, bacteriocins have been the subject of intensive research. In recent years there has in particular been considerable interest in bacteriocins isolated from lactic acid bacteria (LAB), in view of their potential utility in the food and brewing industries, in particular in the preservation of food products.

It is known that short chain (1-3) β-glucans have an immunostimulating effect in salmon. For example, salmon treated with short chain (1-3) β-glucans have been shown to have increased resistance to different types of bacteria including Yersina ruckeri (Enteric redmouth disease), Vibro anguillarum (Classical vibrose) and Vibro salmonicidia (Hitra disease) (see Robertsen B, Rørstad G, Engstad R. and Raa J, Journal of fish diseases 13, 1990, 391-400).

Thus viewed from one aspect the present invention provides an animal feed, comprising a cereal fermented with lactic acid bacteria, wherein said cereal is selected from the group consisting of wheat, oats, rye and barley, together with at least one animal foodstuff component, said feed has been subjected to expansion, compression, extrusion and/or drying.

Preferably the animal feed is a fish (e.g. salmon) feed or bird (e.g. chicken) feed.

Viewed from a further aspect the present invention provides a process for producing an animal feed, said process comprising the steps of:
(a) fermenting a cereal with lactic acid bacteria, said cereal being selected from wheat, barley, rye and oats;
(b) admixing the fermented cereal with at least one animal foodstuff; and, optionally
(c) processing said admixture by expansion, compression, extrusion and/or drying.

Especially preferably a cereal is fermented with a bacteriocin-producing lactobacillus strain, particularly especially preferably a Sakacin A or Sakacin P producing lactobacillus strain.

Viewed from a still further aspect the invention provides the use of fermented cereal, preferably a bacteriocin containing fermented cereal, as a component in processed animal feed, preferably in fish or bird feed.

Viewed from a yet still further aspect the present invention provides a method of achieving an immunostimulatory response in an animal body by administering thereto an animal feed as defined hereinbefore and a method of reducing the level of cholesterol in an animal body by administering thereto an animal feed as defined hereinbefore together with a digestible amount of fibre capable of reducing the cholesterol level in said animal.

Viewed from an even further aspect the present invention provides the use of a fermented cereal in the manufacture of an animal feed for use in reducing the level of cholesterol in the animal to which the feed is fed or for use in a method of achieving an immunostimulatory response in an animal body.

Cereal fermentation has been found to have the advantage of changing the level of mono- and disaccharides present in the cereal and the solubility of polysaccharides in the cereal, allowing the cereal grains to swell and partly dissolve. The admixture may then more easily undergo extrusion and thereby become more digestible for the fish.

Moreover, fermentation yields a feed with a lower pH than identical feed containing unfermented cereal. This is particularly significant in view of the fact that the main pigments used in salmonid feed, astaxanthins, are known to have increased stability under acidic conditions. The pH stabilisation of pigments (carotenoids) in pelletised fish feeds is, therefore, an important advantage of the invention, particularly in view of the high costs of pigments in feed.

A further advantage of the process according to the invention is that it produces animal feed which may exhibit an increased immunostimulating effect in the animal to which it is fed. In this regard, it has been found that fish feed containing fermented barley has an immunostimulating effect in the fish to which it is fed. Without wishing to be bound by any theoretical considerations, it is thought that this effect may be due to the formation of short chain (1-3) β-glucan during cereal fermentation by degradation of polysaccharides such as β-glucan.

Another advantage of animal feed prepared in accordance with the process according to the invention is that fermentation of the cereal provides a fish feed with improved digestibility, particularly with regard to starch digestibility. This in turn allows a greater fibre content to be added to the feed than would otherwise be possible. This is important because fibre is an important dietary requirement for animals and is known to have a cholesterol lowering effect. Typically up to 10% by weight of fibre will be digestible in fish feed according to the invention compared to an upper limit of 2.5% in conventional feed. Preferably the fibre content will be in the range 4-10% by weight.

The feed produced according to the invention may be processed to any conventional form, eg. by expansion, extrusion, heating, drying, or pelleting. By pellets, is meant any form of self-supporting feed unit, eg. tablets, cakes, bricks etc. Pelleting can be effected by compression but most conveniently will be by extrusion. While feeds in pellet form are preferred, the feeds according to the invention may be in other forms, eg. flakes or powders.

The cereal used in the present invention may be any feedstuff cereal. However for fish feed barley, wheat and oats are preferred and oats and barley are especially preferred. The digestibility in salmonids of cereal starch present in fish feed prepared in accordance with the process according to the invention is improved and it has been shown that for barley the increase in digestibility is significant (nearly 100%) and for wheat it is 20%.

The fermented cereal conveniently makes up from 1 to 95%, preferably 10 to 40% by weight of the animal feed. The remaining components may be conventional animal feedstuffs and feedstuff additives, such as unfermented cereals, fish meal, bacteriocins, fats, vitamins, minerals, water, colorants, flavour-enhancers, therapeutic agents, stabilizers, antioxidants and pH regulators. Cereal components may be whole grain, grain fragment, crushed grain or milled grain (eg. flour).

In the case of fish feed, the total cereal content of the feed is preferably no more than about 40% by weight and of this it is preferred that the major part be pre-fermented. Especially preferably substantially all the cereal component is pre-fermented.

The bacteriocins present as components of the fermented cereal may be produced during fermentation as mentioned hereinbefore but may also be produced separately and added pure or in a crude or partly purified form prior to processing.

The bacteriocins used may have one or several inhibition spectra. Preferably bacteriocin having inhibitative effects against pathogen-producing bacteria (such as Listeria) found in feedstuffs will be used.

The cereal fermentation is preferably effected using a lactic acid bacteria and in particular a bacteriocin producing Lactobacillus strain. Many Lactobacilli are known and are commercially available, for example for use in bread baking, eg. from Chr Hanson, Denmark. Preferred fermentation agents include the lactic acid bacteria containing sour dough cultivars.

To inhibit bacterial strains such as Listeria monocytogenes in the animal feed, a bacteriocin producing lactobacillus strain may be used. Several such strains are known, for example L. Sake strain Lb674 and Lb706 producing Sakacin P and Sakacin A respectively (L. Sake Lb674 is available from Dr. Lothar Kröckel, Federal Institute of Meat Research, Kulmach, Germany and L. Sake Lb706 from MATFORSK, The Norwegian Food Research Institute, Osloveien, Norway).

Lactic acid bacteria bacteriocins are usually small peptides, seldom containing more than 60 amino acids. Many bacteriocins from lactic acid bacteria have now been described (for a review see Nettles et al., 1993) and include, most notably, the well-studied nisin, (see for example Gross, et al., J. Am. Chem. Soc. 93: 4634-4635, 1971 and Hurst, Adv. Appl. Microbiol. 27: 85-123, 1981).

Preferably a bacteriocin producing strain should simultaneously carry out lactic acid fermentation and produce bacteriocin. As bacteriocins are peptides, they may be decomposed and inactivated by proteinases in the flour. In such cases it may be preferable to add bacteriocin prior to processing or to pre-heat the flour to inactivate any such proteinases. If the bacteriocin is unable to survive processing (eg. extrusion) then further bacteriocin or bacteriocin producing strains may be added after processing.

The fermentation step in the process according to the invention is conveniently effected in an aqueous medium, generally water, containing 40-60% by weight cereal, preferably in the form of finely ground whole grain flour, for a period of 10 to 48 hours, preferably about 24-48 hours, until an acid pH is obtained, for example a pH of 1-6, especially 3-5, and at ambient or above ambient temperature, for example 25 to 35°C, especially about 30°C.

In the fermentation step, a starter culture may be used or alternatively one may use a spontaneous culture obtained from the cereal used, optionally one obtained in advance. Using a starter culture the fermentation period would typically be 10 to 16 hours, while with spontaneous fermentation a longer period of 24 to 48 hours might be used. The fermentation will not generally be effected using an initially acid medium. The acid pH develops as a result of acid fermentation during fermentation.

The following Examples are intended to illustrate the invention in a non-limiting manner:

### EXAMPLE 1

The raw materials used to carry out the following experiments were:
(a) Wheat - whole grain wheat flour;
(b) Oats - finely ground whole grain oat flour;
(c) Fish meal - Norseamink, Norwegian fish meal with antioxidant, Egersund Sildoljefabrikk, Norway.

Four mixtures were prepared by admixing fish meal with the following untreated or pre-treated cereals:
(1) Untreated wheat (15%)
(2) Untreated oats (15%)
(3) Fermented oats (sour-dough) - (based on 15% flour weight)
(4) Scalded oats (based on 15% flour weight)

Fish feed mixtures containing cereals (1) and (2) were prepared by manually mixing the untreated wheat or oat flour (15%) with fish meal. The water content for both mixtures was 8.1%.

For the pre-treatment of the fish feed mixture containing (3), fermentation was carried out by adding equal amounts of oats flour and water to an equivalent amount of lactic acid bacteria (sour dough) culture. The mixture (20°C, pH 4.6) was fermented for 24 hours at 30°C to produce a preculture. The preculture was refreshed regularly with oat flour and water prior to extrusion a week later. The pH of the final fermentation mixture was 4.0 and when admixed with fish meal yielded a mixture with a water content of 19.9%.

For the pre-treatment of the fish feed mixture containing (4), boiling water was added to oats flour in a 1:1 ratio. Shortly after addition, the temperature was 76°C and cooling to 30°C led to a weight loss of 3%. The scalded flour/water mixture was added to fish meal yielding a mixture with a water content of 19.9%.

Extrusion was carried out on a Werner & Pleider, Continua 37 extruder. The temperature of the extruder was in the range 130-150°C and water was added at a rate of between 2 and 7.5 l/hour. Further variants included the rotating screw (280-320 rpm), the flour feeding rate (1.2-2.4 g/min) and the feeding temperature (130-145°C). During the extrusion process, the pressure varied between 10 and 25 bar.

The pellets were allowed to cool and dry at ambient temperature and then packed in closed drums. For each experiment at least two fractions were sampled and the results therefrom are compiled in Tables 1 and 2 below.

The % dry matter for each sample was determined shortly after production and again after 24 hours in air at ambient temperature. The pH was also measured. Breakage and crushing strength were measured in a Kramer cell in an Instron Universal testing machine. The breakage strength was measured perpendicular to the length of the pellet and averaged over 12 pellets. The width and length of each pellet were measured together with their water solubility and rate of sinking. As used herein, the term solubility refers to the % weight of pellets dissolved in water during a certain period of soaking in water.

Results of the tests carried out on the 4 types of fish feed mixture are presented in Tables 1 and 2 below.

**Table 1**

| Dry matter (%) and pH of pellets from wheat and oats | | | |
|---|---|---|---|
| Treatment | Dry matter (%) at production | after 24 h | pH |
| Untreated wheat | 81.9 | 89.9 | 6.1 |
| Untreated oats | 77.1 | 87.3 | 6.0 |
| Fermented oats | 75.6 | 87.7 | 5.8 |
| Scalded oats | 76.4 | 86.6 | 6.0 |

**Table 2**

| Breakage and water solubility of pellets from wheat and oats | | | | |
|---|---|---|---|---|
| Treatment | Breakage | Crushing | Water solubility (%) | |
| | N | N | 2 min | 20 h |
| Untreated wheat | 16.4 | 213 | 8 | 62 |
| Untreated oats | 16.6 | 103 | 15 | 38 |
| Fermented oats | 16.4 | 127 | 9 | 32 |
| Scalded oats | 16.3 | 134 | 19 | 100 |

Notably from the results in Table 1, drying for 24 hours at ambient temperature led to an 8 to 12.1% increase in the dry matter content with the most extensive drying observed in the fermented oats. All of the pellets proved to be stable toward mould growth or any other visible deterioration over a 12 month period. In addition, pellets containing fermented oats had a lower pH than the other pellet-types.

Whilst breakage strength showed no significant variation amongst the pellet-types, a greater force was required to crush the wheat-containing pellets than any of the oat-containing pellets.

Water solubility was determined from the time taken for pellets to sink in water. All pellet types took several hours to sink but when finally wetted, solubility rose.

### EXAMPLE 2

### Experiments in Atlantic salmon fed with feed containing different amounts of fermented and unfermented wheat or barley flour

The following describes the utility of animal feed produced by the process according to the invention. Feed composition is stated alongside digestibility results, carbohydrate analysis and immunostimulating effects in salmon. In all experiments, the salmon performed well.

The raw materials used to carry out the following experiments were:
(a) Wheat - whole grain wheat flour, finely ground;
(b) Barley - whole grain barley flour, coarsely ground.

Eight mixtures were prepared by admixing fish meal with the following pre-treated (fermented) or untreated (unfermented) cereals:

| | | | |
|---|---|---|---|
| (1) | Pretreated wheat | (12%) | (HF 12) |
| (2) | Pretreated wheat | (24%) | (HF 24) |
| (3) | Pretreated wheat | (36%) | (HF 36) |
| (4) | Untreated wheat | (24%) | (HU 24) |
| (5) | Pretreated barley | (12%) | (BF 12) |
| (6) | Pretreated barley | (24%) | (BF 24) |
| (7) | Pretreated barley | (36%) | (BF 36) |
| (8) | Untreated barley | (24%) | (BU 24) |

The feed compositions were chosen to give equal levels of energy and are shown in Table 3. Extrusion conditions were as described above in Example 1.

**Table 3**

| Feed compositions (kg/100 kg feed) | | | | |
|---|---|---|---|---|
| Type of feed ingredient | Fermented | | | Unfermented |
| | 1 | 2 | 3 | 4 |
| Coding | H(B)F 12 | H(B)F 24 | H(B)F 36 | H(B)U24 |
| Wholemeal wheat/barley | 12 | 24 | 36 | 24 |
| Fish meal | 66 | 57 | 48 | 57 |
| Fish oil | 22 | 19 | 16 | 19 |
| Sum | 100 | 100 | 100 | 100 |
| Indicator (Ytriumoxide) | 100 mg/kg feed | | | |
| Mineral mix | 100 mg/kg feed | | | |
| Astaxanthin | 75 ppm (937.5 mg Carophyll Pink/kg feed) | | | |

The feed was fed *ad libitum* to Atlantic salmon weighing 0.5 kg. After an adaption period of 9 days, the experimental period lasted for 3 weeks. Faeces were collected on day 8 and day 16 according to the method of Austreng, E. (see Aquaculture, 13: 265-272, 1978). Faeces were freeze-dried prior to analysis.

### Chemical composition of feed

Chemical composition of feed as revealed by analysis is given in Table 4.

**Table 4**

| Chemical composition of feed (g/100g feed) | | | | | | |
|---|---|---|---|---|---|---|
| Type of feed | Dry matter | Protein | Fat | Starch | betaglucan | sugars (mono- and di) |
| Wheat | | | | | | |
| HF 12 | 93.7 | 47.4 | 28.5 | 5.6 | 0.05 | 0.21 |
| HF 24 | 93.7 | 43.0 | 25.8 | 11.5 | 0.08 | 0.48 |
| HF 36 | 93.1 | 39.0 | 21.8 | 17.2 | 0.07 | 0.67 |
| HU 24 | 94.0 | 42.2 | 26.2 | 11.1 | 0.08 | 0.20 |
| | | | | | | |

| Barley | | | | | | |
|---|---|---|---|---|---|---|
| BF 12 | 94.4 | 47.3 | 28.9 | 4.5 | 0.28 | 0.11 |
| BF 24 | 94.1 | 42.5 | 24.8 | 10.2 | 0.53 | 0.38 |
| BF 36 | 93.6 | 38.0 | 22.0 | 15.3 | 0.79 | 0.59 |
| BU 24 | 94.2 | 42.8 | 26.2 | 8.2 | 0.40 | 0.00 |

### Effects of fermentation on digestibility

Digestibility (as %) of protein, fat, starch and energy were calculated from chemical analysis of feed and faeces collected from the fish. The results are given in Table 5.

**Table 5**

| Digestibility (%) of fish feed fed to Atlantic salmon | | | | |
|---|---|---|---|---|
| Type of feed | Protein | Fat | Starch | Energy |
| Wheat | | | | |
| HF 12 | 83.3 | 88.7 | 70.9 | 83.6 |
| HF 24 | 84.3 | 89.6 | 57.2 | 81.6 |
| HF 36 | 85.5 | 84.5 | 38.4 | 75.8 |
| HU 24 | 84.3 | 86.5 | 47.9 | 79.8 |
| | | | | |

| Barley | | | | |
|---|---|---|---|---|
| BF 12 | 84.4 | 85.4 | 68.9 | 81.7 |
| BF 24 | 85.7 | 86.9 | 63.2 | 81.1 |
| BF 36 | 85.8 | 84.4 | 48.0 | 75.4 |
| BU 24 | 84.6 | 87.3 | 33.2 | 80.2 |

For protein, there was a tendency for increased digestibilty with increasing amounts of fermented wheat or barley flour in the fish feed. For feed containing barley, there was a slightly higher protein digestibility when the flour was fermented (85.7) compared with non-fermented feed (84.6). These feeds were both formulated with 24 % barley flour.

For fats, no systematic variation in % digestibilty due to carbohydrate level or treatment were seen.

For starch digestibility, significant positive effects of fermentation were obtained for both wheat- and barley-containing feeds. In feeds containing 24% fermented wheat flour, starch digestibility was 57.2% compared with 47.9% for unfermented feed. This corresponds to an increase in starch digestibility of 19%.

For barley the effect of starch digestibility due to fermentation was even more pronounced; non-fermented feed with 24% flour had a digestibility of only 33.2% while that of the fermented feed was 63.2%. This is an increase in starch digestibility of more than 90%.

As is known from previous research, starch digestibility decreases with increased levels of cereals. This was also seen in the present experiments. However, for barley the fermentation process compensated for this so that the starch digestibility of feed containing 36% fermented barley (48.0%) was higher than the digestibility of feed containing the lower level of 24% unfermented barley (33.2%).

The increased digestibility of starch due to fermentation was supported by the results for energy digestibility. The increased digestibility of fermented samples caused higher energy digestibility than the unfermented samples. For wheat containing 24% flour the digestibilities were 81.6% and 79.8% for fermented and unfermented feed respectively. For barley, the corresponding figures were 81.1% and 80.2%.

### Effects of fermentation on fish performance

The salmon performed well during the experimental period with no problems with appetite in the fish. Faeces were of normal consistency, even at the higher carbohydrate levels. This is reflected in normal dry matter content of the faeces, with no sign of diarrhoea (dry matter below 12).

| Type of feed | Dry matter in faeces (g/100g) |
|---|---|
| Wheat | |
| HF 12 | 15.0 |
| HF 24 | 14.4 |
| HF 36 | 15.7 |
| HU 24 | 14.3 |
| | |

| Barley | |
|---|---|
| BF 12 | 14.9 |
| BF 24 | 13.6 |
| BF 36 | 17.2 |
| BU 24 | 15.3 |

### Effects of fermentation on carbohydrate composition

The solubilty of carbohydrates in the feed was influenced by the fermentation process. Water soluble carbohydrate contents of wheat and barley flour prior to and after fermentation are given in Table 6.

**Table 6**

| Carbohydrates (g/100g feed) in wheat and barley flour prior to and after fermentation | | | | | |
|---|---|---|---|---|---|
| Sample | Glucose (G) | Maltose (M) | Water soluble polysaccharide | Total G+M | Total |
| Unfermented | | | | | |
| Wheat | 0.72 | 5.72 | 2.05 | 6.44 | 8.49 |
| Barley | 1.05 | 4.81 | 5.61 | 5.86 | 11.47 |
| | | | | | |

| Fermented | | | | | |
|---|---|---|---|---|---|
| Wheat | 2.04 | 1.16 | 1.24 | 3.20 | 4.44 |
| Barley | 2.00 | 0.06 | 2.76 | 2.06 | 4.82 |

As these results show, the content of maltose decreased considerably, whilst the amount of glucose increased during fermentation. The amount of mono- and disaccharides was nearly half that of the unfermented flour for both wheat and barley. Also the amount of water soluble polysaccharides decreased during the fermentation process. Approximately half as much water soluble polymeric compounds was detected after fermentation. The lowering in soluble polysaccharides was most extensive in barley flour where the initial level of 11.5% was lowered to 4.8%, a reduction of 59%.

### Immunostimulating effects

Experimental fish feed was fed to salmon for 16 days and blood samples were tested for serum lyzoyme activity and used to evaluate the immunostimulating effect in the blood. The results are shown in Table 7.

**Table 7**

| Serum lysozyme activity in salmon fed feed containing fermented and unfermented barley | |
|---|---|
| Type of feed | Lysozyme activity (%) |
| Barley | |
| BF 12 | 28.445^{abc}* |
| BF 24 | 32.852^{a} |
| BF 36 | 28.480^{abc} |
| BU 24 | 23.004^{c} |

| | |
|---|---|
| * figures denoted with the same letter are not significantly different (P<0.5) | |

Serum lysozyme activity was determined by the Micrococcus lysoplate assay. In this method the sample is placed in a small well stamped out in an agarose gel containing the bacteria Micrococcus lysodeicticus. After incubation, the agarose gels are stained and destained and the diameter of the lysed zones are measured. The results are compared with the diameter of a reference serum (100%) known to contain lysozyme activity and given as a percent of the diameter of the reference. Actual levels of lysozymes may vary between groups of fish due to health status, age etc. The effect in a special trial is evaluated as the relative lysozyme activities of fish within that trial.

Lysozyme is an enzyme which hydrolyses cell wall components of bacteria. By degradation of the cell walls, the growth of the bacteria is inhibited. High lysozyme activity may therefore give an increased resistance toward pathogen bacteria and thereby enhanced health to the animal.

The results clearly demonstrate increased levels of lysozyme activity in salmon fed with fermented barley compared with salmon fed with unfermented feed. Lysozyme activity was significantly higher in salmon fed with 24% fermented barley (32.85%) compared with salmon fed with the same amount of unfermented barley (23.00%).

## Claims

1. An animal feed comprising a cereal fermented by lactic acid bacteria together with at least one animal feedstuff component, said cereal being selected from the group consisting of wheat, oats, rye and barley, wherein said feed has been subjected to expansion, compression, extrusion and/or drying.

2. A feed as claimed in claim 1 wherein said cereal is grain fragment, crushed grain or milled grain.

3. A feed as claimed in any preceding claim wherein said cereal is oats or barley.

4. A feed as claimed in any preceding claim having an improved digestibility over its non-fermented cereal-containing animal feed analogue.

5. A feed as claimed in claim 4 having improved starch digestibility over its non-fermented cereal-containing animal feed analogue.

6. A feed as claimed in any preceding claim wherein the animal feedstuff component is selected from unfermented cereals, bacteriocin, fish meal and pigment.

7. A feed as claimed in any preceding claim comprising one or more bacteriocins.

8. A feed as claimed in claim 7 wherein the bacteriocin has an inhibitory effect against pathogen producing bacteria.

9. A feed as claimed in claim 8 wherein said bacteria is a species of listeria.

10. A feed as claimed in any preceding claim comprising an amount of short chain (1-3) β-glucan capable of achieving an immunostimulatory response.

11. A feed as claimed in any preceding claim comprising an amount of fibre capable of having a cholesterol reducing effect.

12. A feed as claimed in any preceding claim wherein said feed is in the form of tablets, cakes, bricks, pellets, flakes or powders.

13. A feed as claimed in any preceding claim wherein fermentation is carried out in an aqueous medium containing 40-60% by weight cereal.

14. A feed as claimed in any preceding claim wherein fermentation is carried out for 10-48 hours.

15. A feed as claimed in any preceding claim wherein fermentation is carred out at a temperature of 25-35°C.

16. A process for producing an animal feed comprising the steps of:
(a) fermenting a cereal with a lactic acid bacteria, said cereal being selected from wheat, barley, rye and oats;
(b) admixing the fermented cereal with at least one animal feedstuff; and
(c) processing said admixture by expansion, compression, extrusion and/or drying.

17. A process as claimed in claim 16 wherein the feed is in the form of tablets, cakes, bricks, pellets, flakes or powders.

18. A process as claimed in claim 16 or claim 17 wherein said lactic acid bacteria is a bacteriocin-producing strain of lactobacillus bacteria.

19. A process as claimed in claim 18 wherein said bacteriocin-producing lactobacillus strain is a Sakacin A or Sakacin P producing lactobacillus strain.

20. A process as claimed in any of claims 16 to 19 wherein bacteriocins or bacteriocin-producing bacterial strains are added in a further step.

21. A process as claimed in claim 20 wherein bacteriocins are added to the admixture prior to or after step (b).

22. A process as claimed in any of claims 18 to 21 wherein the bacteriocin has an inhibitory effect against pathogen producing bacteria.

23. A process as claimed in claim 22 wherein said bacteria is a species of listeria.

24. A process as claimed in any one of claims 16 to 23 wherein fermentation is carried out in an aqueous medium containing 40-60% by weight cereal.

25. A process as claimed in any one of claims 16 to 24 wherein fermentation is carried out for 10-48 hours.

26. A process as claimed in any one of claims 16 to 25 wherein fermentation is carred out at a temperature of 25-35°C.

27. Use of a cereal fermented by lactic acid bacteria in the manufacture of an animal feed as described in any one of claims 1 to 26 for use in reducing the level of cholesterol in the animal to which the feed is fed.

28. Use of a cereal fermented by lactic acid bacteria in the manufacture of an animal feed as described in any one of claims 1 to 26 for use in a method of achieving an immunostimulatory response in an animal body.

## Patentansprüche

1. Tierfutter, das ein durch Milchsäurebakterien fermentiertes Getreide zusammen mit mindestens einer Tierfuttermittelkomponente aufweist, wobei das Getreide aus der aus Weizen, Hafer, Roggen und Gerste bestehenden Gruppe ausgewählt ist, und wobei das Futter einer Expansion, Kompression, Extrusion und/oder Trocknung unterzogen wurde.

2. Futter nach Anspruch 1, wobei das Getreide Kornbruch, zerstoßenes Korn oder gemahlenes Korn ist.

3. Futter nach einem der vorstehenden Ansprüche, wobei das Getreide Hafer oder Gerste ist.

4. Futter nach einem der vorstehenden Ansprüche, das eine verbesserte Verdaubarkeit gegenüber vergleichbarem nicht-fermentiertem getreidehaltigem Tierfutter aufweist.

5. Futter nach Anspruch 4, das eine verbesserte Stärkeverdaubarkeit gegenüber vergleichbarem nicht-fermentiertem getreidehaltigem Tierfutter aufweist.

6. Futter nach einem der vorstehenden Ansprüche, wobei die Tierfuttermittelkomponente aus nicht-fermentierten Getreiden, Bakteriozin, Fischfutter und Pigment ausgewählt ist.

7. Futter nach einem der vorstehenden Ansprüche, das eines oder mehrere Bakteriozine aufweist.

8. Futter nach Anspruch 7, wobei das Bakteriozin eine Hemmwirkung gegen Krankheitserreger bzw. pathogene Stoffe herstellende Bakterien aufweist.

9. Futter nach Anspruch 8, wobei die Bakterien eine Listerien-Spezies sind.

10. Futter nach einem der vorstehenden Ansprüche, das eine Menge von kurzkettigem (1-3)-β-Glukan aufweist, das dazu in der Lage ist, eine immunstimulierende Reaktion zu erreichen.

11. Futter nach einem der vorstehenden Ansprüche, das eine Menge von Fasern bzw. Ballaststoffen aufweist, die dazu in der Lage sind, eine cholesterinreduzierende Wirkung zu haben.

12. Futter nach einem der vorstehenden Ansprüche, wobei das Futter in der Form von Tabletten, Kuchen, Blöcken, Pellets, Flocken oder Pulver vorliegt.

13. Futter nach einem der vorstehenden Ansprüche, wobei die Fermentation in einem 40 - 60 Gew.-% Getreide enthaltenden wässrigen Medium durchgeführt wird.

14. Futter nach einem der vorstehenden Ansprüche, wobei die Fermentation über 10 - 48 Stunden geführt wird.

15. Futter nach einem der vorstehenden Ansprüche, wobei die Fermentation bei einer Temperatur von 25 - 35 °C geführt wird.

16. Verfahren zur Herstellung eines Tierfutters, das die folgenden Schritte aufweist:
(a) Fermentieren eines Getreides mit einem Milchsäurebakterium, wobei das Getreide aus Weizen, Gerste, Roggen und Hafer ausgewählt ist,
(b) Mischen des fermentierten Getreides mit mindestens einem Tierfuttermittel, und
(c) Verarbeiten der Mischung durch Expansion, Kompression, Extrusion und/oder Trockung.

17. Verfahren nach Anspruch 16, wobei das Futter in der Form von Tabletten, Kuchen, Blöcken, Pellets, Flocken oder Pulver vorliegt.

18. Verfahren nach Anspruch 16 oder Anspruch 17, wobei das Milchsäurebakterium ein Bakteriozin herstellender Stamm von Laktobacillus-Bakterien ist.

19. Verfahren nach Anspruch 18, wobei der Bakteriozin herstellende Laktobacillus-Stamm ein Sakacin A oder Sakacin P herstellender Laktobazillus-Stamm ist.

20. Verfahren nach einem der Ansprüche 16 bis 19, wobei Bakteriozine oder Bakteriozin herstellende Bakterienstämme in einem weiteren Schritt zugegeben werden.

21. Verfahren nach Anspruch 20, wobei Bakteriozine der Mischung vor oder nach Schritt (b) zugegeben werden.

22. Verfahren nach einem der Ansprüche 18 bis 21, wobei das Bakteriozin eine Hemmwirkung gegen pathogene Stoffe herstellende Bakterien aufweist.

23. Verfahren nach Anspruch 22, wobei das Bakterium eine Listerien-Spezies ist.

24. Verfahren nach einem der Ansprüche 16 bis 23, wobei die Fermentation in einem 40 - 60 Gew.-% Getreide enthaltenden wässrigen Medium durchgeführt wird.

25. Verfahren nach einem der Ansprüche 16 bis 24, wobei die Fermentation über 10 - 48 Stunden geführt wird.

26. Verfahren nach einem der Ansprüche 16 bis 25, wobei die Fermentation bei einer Temperatur von 25 - 35 °C geführt wird.

27. Verwendung eines durch Milchsäurebakterien fermentierten Getreides bei der Herstellung eines Tierfutters nach einem der Ansprüche 1 bis 26 für die Verwendung bei der Reduktion des Cholesterinwertes bei dem Tier, dem das Futter verfüttert wird.

28. Verwendung eines durch Milchsäurebakterien fermentierten Getreides bei der Herstellung eines Tierfutters nach einem der Ansprüche 1 bis 26 für die Verwendung in einem Verfahren zur Erreichung einer immunstimulierenden Reaktion in einem Tierkörper.

## Revendications

1. Aliment pour animaux, comprenant une céréale fermentée par des bactéries lactiques, conjointement avec au moins un composant d'aliment pour animaux, ladite céréale étant choisie dans l'ensemble constitué par le blé, l'avoine, le seigle et l'orge, ledit aliment ayant été soumis à une expansion, une compression, une extrusion et/ou un séchage.

2. Aliment selon la revendication 1, dans lequel ladite céréale est sous forme de fragment de grain, de grain concassé ou de grain moulu.

3. Aliment selon l'une quelconque des revendications précédentes, dans lequel ladite céréale est l'avoine ou l'orge.

4. Aliment selon l'une quelconque des revendications précédentes, ayant une digestibilité améliorée par rapport à son analogue aliment pour animaux contenant une céréale non fermentée.

5. Aliment selon la revendication 4, ayant une digestibilité d'amidon améliorée par rapport à son analogue aliment pour animaux contenant une céréale non fermentée.

6. Aliment selon l'une quelconque des revendications précédentes, dans lequel le composant d'aliment pour animaux est choisi parmi des céréales non fermentées, la bactériocine, la farine de poisson et un pigment.

7. Aliment selon l'une quelconque des revendications précédentes, comprenant une ou plusieurs bactériocines.

8. Aliment selon la revendication 7, dans lequel la bactériocine a un effet inhibiteur sur des bactéries productrices d'agents pathogènes.

9. Aliment selon la revendication 8, dans lequel lesdites bactéries appartiennent à une espèce de *Listeria*.

10. Aliment selon l'une quelconque des revendications précédentes, comprenant une quantité de (1-3)β-glucane à courte chaîne capable de produire une réponse immunostimulatrice.

11. Aliment selon l'une quelconque des revendications précédentes, comprenant une quantité de fibres capables d'avoir une effet hypocholestérolémiant.

12. Aliment selon l'une quelconque des revendications précédentes, dans lequel ledit aliment est sous la forme de comprimés, gâteaux, briques, granules, flocons ou poudre.

13. Aliment selon l'une quelconque des revendications précédentes, dans lequel la fermentation est effectuée dans un milieu aqueux contenant 40-60 % en poids de céréale.

14. Aliment selon l'une quelconque des revendications précédentes, dans lequel la fermentation est effectuée pendant 10-48 heures.

15. Aliment selon l'une quelconque des revendications précédentes, dans lequel la fermentation est effectuée à une température de 25-35°C.

16. Procédé pour la production d'un aliment pour animaux, comprenant les étapes suivantes :
(a) fermentation d'une céréale avec une bactérie lactique, ladite céréale étant choisie parmi le blé, l'orge, le seigle et l'avoine ;
(b) mélange de la céréale fermentée avec au moins un composant d'aliment pour animaux ; et
(c) élaboration dudit mélange par expansion, compression, extrusion et/ou séchage.

17. Procédé selon la revendication 16, dans lequel l'aliment est sous la forme de comprimés, gâteaux, briques, granules, flocons ou poudre.

18. Procédé selon la revendication 16 ou 17, dans lequel ladite bactérie lactique est une souche de *Lactobacillus* productrice de bactériocine.

19. Procédé selon la revendication 18, dans lequel ladite souche de *Lactobacillus* productrice de bactériocine est une souche de *Lactobacillus* productrice de sakacine A ou sakacine B.

20. Procédé selon l'une quelconque des revendications 16 à 19, dans lequel les bactériocines ou souches bactériennes productrices de bactériocine sont ajoutées dans une étape ultérieure.

21. Procédé selon la revendication 20, dans lequel les bactériocines sont ajoutées au mélange avant ou après l'étape (b).

22. Procédé selon l'une quelconque des revendications 18 à 21, dans lequel la bactériocine a un effet inhibiteur sur des bactéries productrices d'agents pathogènes.

23. Procédé selon la revendication 22, dans lequel ladite bactérie appartient à une espèce de *Listeria*.

24. Procédé selon l'une quelconque des revendications 16 à 23, dans lequel la fermentation est effectuée dans un milieu aqueux contenant 40-60 % en poids de céréale.

25. Procédé selon l'une quelconque des revendications 16 à 24, dans lequel la fermentation est effectuée pendant 10-48 heures.

26. Procédé selon l'une quelconque des revendications 16 à 25, dans lequel la fermentation est effectuée à une température de 25-35°C.

27. Utilisation d'une céréale fermentée par des bactéries lactiques, dans la fabrication d'un aliment pour animaux tel que décrit dans l'une quelconque des revendications 1 à 26, pour abaisser le taux de cholestérol chez l'animal auquel est donné l'aliment.

28. Utilisation d'une céréale fermentée par des bactéries lactiques, dans la fabrication d'un aliment pour animaux tel que décrit dans l'une quelconque des revendications 1 à 26, dans un procédé d'obtention d'une réponse immunostimulatrice dans le corps d'un animal.
